# EUROPÄISCHE PATENTANMELDUNG

(11) **EP 0 603 618 A2**
(43) Veröffentlichungstag der Anmeldung: **29.06.1994**
(21) Anmeldenummer: 93119579.6
(22) Anmeldetag: 04.12.1993
(51) Int. Cl.: G01N 33/00, G01M 15/00, G01M 17/00

(54) **Erfassung der Verdampfungsemission eines Fahrzeuges**

(30) Priorität: 19.12.1992 DE 4243250
(71) Anmelder: York International GmbH, D-68165 Mannheim (DE)
(72) Erfinder: Bellstedt, Michael, Dr., D-67346 Speyer (DE); Plapper, Helmut, D-68549 Ilvesheim (DE); Rupp, Heinz, D-68542 Heddesheim (DE)
(74) Vertreter: Rupprecht, Klaus, Dipl.-Ing.

(57) **Zusammenfassung**

Die Anmeldung betrifft ein Verfahren zur Erfassung der Verdampfungsemission eines Fahrzeugs (18), insbesondere zur Erfassung dessen Emission von Kohlenwasserstoffverbindungen, sowie eine Vorrichtung zur Durchführung des Verfahrens, wobei das Fahrzeug (18) in einem gasdicht abschließbaren Raum (10) abgestellt wird, und die im Raum (10) befindliche Luft entsprechend einer vorgegebenen Temperatur-Zeit-Kurve zyklisch erwärmt und abgekühlt wird und die während dieser Zyklen emittierten Kohlenwasserstoffverbindungen qualitativ und quantitativ bestimmt werden. Dabei wird der Innendruck in dem Raum (10) auf Außendruckniveau gehalten.

## Beschreibung

Die Erfindung betrifft ein verfahren zur Erfassung der Verdampfungsemission eines stehenden Fahrzeugs, insbesondere zur Erfassung dessen Emission von Kohlenwasserstoffen sowie eine Vorrichtung zur Durchführung des Verfahrens.

Es ist allgemein bekannt, daß in Kraftfahrzeugen im zunehmenden Maße Kunststoffe zum Einsatz gelangen, um hierdurch Gewichtsvorteile zu erreichen. Dabei werden sowohl Formteile im Fahrzeuginneren, z. B. Front- und Seitenverkleidung sowie der Dachhimmel, als auch technische Komponenten, z. B. Kraftstoffbehälter und Leitungen, Luftfiltergehäuse sowie die Isolation von elektrischen Kabeln, aus Kunststoffen hergestellt.

Ferner ist bekannt, daß bestimmte in Kunststoffen gebundene Kohlenwasserstoffverbindungen, z. B. Weichmacher, sowie in Anstrichstoffen enthaltene Lösungsmittel mit der Zeit aus ihrem Trägermaterial entweichen. Aus Gründen des Umweltschutzes besteht allgemein das Bestreben, derartige Emissionen soweit wie möglich einzuschränken, um so den Gehalt an Kohlenwasserstoffverbindungen in der Luft nicht noch weiter ansteigen zu lassen. Hierfür verbindliche Vorschriften sind in Vorbereitung, wobei darin bestimmte Grenzwerte für die zeit- und temperaturabhängige Emission von Kohlenwasserstoffverbindungen aus Fahrzeugen festgelegt werden sollen.

Einhergehend mit dieser Grenzwertfestlegung ergibt sich die Notwendigkeit, die Einhaltung dieser Vorschrift überprüfen zu können. Hierzu ist es erforderlich, eine Möglichkeit zu schaffen, durch welche der Verlauf der Emission an Kohlenwasserstoffverbindungen über der Zeit in Abhängigkeit von der jeweiligen Umgebungstemperatur störungsfrei und unverfälscht erfaßt werden kann.

Es ist bereits versucht worden, die Einstellung von vom Umgebungsdruck unabhängigen Prüfbedingungen in einem Prüfraum dadurch zu erreichen, daß in dem Prüfraum, ein einziger größenveränderlicher Beutel angeordnet wird und dieser mit einer bestimmten Luftmenge befüllt wird. Dies erfolgt durch Messung der Luftmenge bzw. des Luftgewichtes. Auf diese Weise sollte eine gewisse Konstanz der Versuchsbedingungen erreicht und das Ausgangsvolumen der Kammer bestimmt werden. Es ist jedoch bekannt, daß Mengenmeßeinrichtungen für gasförmige Stoffe vergleichsweise ungenau und zudem sehr kostspielig sind. Zuverlässige Aussagen lassen sich mit dieser Prüfanordnung nicht ohne weiteres herleiten. Das für die Messung der Emission zu bestimmende Ausgangsvolumen der Kammer kann mit dieser Einrichtung nicht zuverlässig ermittelt werden.

Ausgehend von diesem Stand der Technik ist es Aufgabe der Erfindung, ein Verfahren der eingangs genannten Art anzugeben, das eine kostengünstige Möglichkeit bietet, den zuvor charakterisierten Emissionsverlauf zu erfassen. Ferner soll eine Vorrichtung angegeben werden, mit welcher das erfindungsgemäße Verfahren durchgeführt werden kann.

Diese Aufgabe wird durch die kennzeichnenden Merkmale des Patentanspruchs 1 gelöst. Danach ist vorgesehen, daß das zu prüfende Fahrzeug in einem gasdicht verschließbaren Raum abgestellt wird, daß entsprechend einer vorgegebenen Zeit-Temperatur-Kurve die im Raum befindliche Luft zyklisch erwärmt und abgekühlt wird, daß die während dieser Zyklen emittierten Kohlenwasserstoffverbindungen qualitativ und quantitativ bestimmt werden und daß der Innendruck in dem Raum gleich dem Außendruck gehalten wird.

In vorteilhafter Weiterbildung der Erfindung ist vorgesehen, daß die Anpassung des Innendrucks an den Außendruck durch Volumenänderung des Raumvolumens erfolgt. Dabei kann insbesondere eine differenzierte Druckanpassung vorgesehen sein, welche einerseits für eine erwärmungsbedingte Druckerhöhung infolge der Erwärmung der Innenluft durch Vergrößerung des verfügbaren Raumvolumens erreicht wird und andererseits für eine durch Erhöhung des Umgebungsdrucks der Atmosphäre hervorgerufene Druckerhöhung in dem Raum durch Verkleinerung des verfügbaren Raumvolumens bewirkt wird.

Gemäß einer bevorzugten Ausführungsform der Erfindung erfolgt die zur Druckkompensation vorgesehene Volumenänderung dadurch, daß ein veränderlicher Teil des Raumvolumens abgetrennt wird und entsprechend dem jeweiligen Druck der Umgebungsatmosphäre vergrößert oder verkleinert wird. Dies erfolgt vorzugsweise mittels eines weiteren abgetrennten Volumenteils des Raumvolumens, der mit der Außenatmosphäre in Verbindung steht.

Eine Vorrichtung zur Durchführung des erfindungsgemäßen Verfahrens ist in Patentanspruch 4 gekennzeichnet. Demgemäß weist ein gasdicht abschließbarer Raum zur Aufnahme eines Fahrzeugs eine Heiz- und Kühlvorrichtung zur Einstellung eines bestimmten Temperatur-Zeit-Verlaufs sowie eine Gaserfassungseinrichtung auf. Darüberhinaus ist in dem Raum eine hiervon gasdicht abgetrennte erste Kompensationskammer angeordnet, welche mit der Umgebungsatmosphäre in Verbindung steht, sowie eine zweite Kammer, die einen verschließbaren Zugang zum Inneren des Raumes aufweist. Statt der horizontalen Nebeneinanderanordnung der Kompensationskammern können die Kammern auch an den Seiten oder an der Rückseite des Prüfraumes angeordnet sein.

In zweckmäßiger Weiterbildung sind beide Kammern, nämlich die erste gasdicht gegen den Innenraum abgetrennte Kompensationskammer wie auch die zweite Kammer jeweils mit einem Ventilator ausgestattet, über welche die vorgesehene Verbindung mit der zugeordneten Umgebungsluft erfolgt.

Gemäß einer bevorzugten Ausführungsform der Erfindung sind beide Kammern als flexible Beutel ausgebildet, deren Volumen aufgrund der Flexibilität variabel einstellbar ist.

Diese und weitere vorteilhafte Ausgestaltungen der Erfindung sind Gegenstand der Unteransprüche.

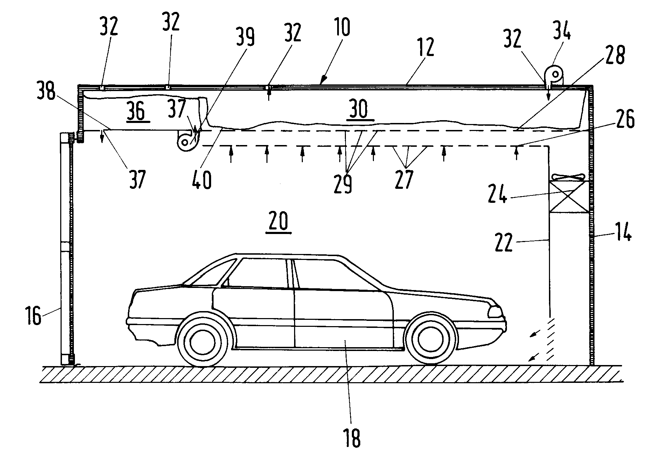

Anhand eines in der Zeichnung dargestellten Ausführungsbeispiels der Erfindung sollen die Erfindung, vorteilhafte Ausgestaltungen und besondere Vorteile der Erfindung näher erläutert und beschrieben werden.

Es zeigt
- die einzige Figur:: eine erfindungsgemäße Vorrichtung mit einem Raum zur Aufnahme eines Fahrzeuges.

In der einzigen Figur ist ein zur erfindungsgemäßen Vorrichtung gehöriger gasdicht abgeschlossener Raum 10 mit einer Decke 12, einer Stirnwand 14 und einem Tor 16 dargestellt, in welchem ein Fahrzeug 18 angeordnet ist. Im Innenraum 20 des Raums 10, in welchem das Fahrzeug 18 untergestellt ist, befindet sich eine an der Stirnwand 14 angeordnete, durch eine Zwischenwand 22 verdeckte Heiz-und Kühleinrichtung 24 mit einem Spülluftventilator 25, welche die Luft im Inneren 20 des Raumes 10 umwälzt und hierbei entsprechend einem vorgegebenen Temperatur-Zeit-Verlauf aufheizt oder abkühlt sowie eine nicht näher dargestellte Gaserfassungseinrichtung. Die Zwischenwand 22 setzt sich unterhalb der Decke 12 parallel hierzu als Zwischendecke 26 fort, wobei die Zwischendecke 26 in regelmäßiger Anordnung mit Durchgangsöffnungen 27 versehen ist, durch welche die Luft aus dem Innenraum 20 hindurchtreten kann.

Oberhalb der Zwischendecke 26 befindet sich in gewissem Abstand eine zweite Zwischendecke 28, die ebenfalls Durchgangsöffnungen 29 aufweist, ebenfalls für den Durchtritt von Luft, und mit der ersten Zwischendecke 26 einen Luftkanal bildet.

Oberhalb der zweiten Zwischendecke 28 ist eine von einem Beutel aus flexiblem Material gebildete erste Kompensationskammer 30 angeordnet, welche gegen den Innenraum 20 gasdicht abgetrennt ist und über Öffnungen 32 mit der Außenatmosphäre in Verbindung steht. Die erste Kompensationskammer 30 ist aus einer flexiblen Folie gebildet, welche die Kammerwand 40 bildet. Um das Anpassen der Kammerwand 40 an die Konfiguration der ersten Kompensationskammer 30 zu erreichen, dient ein Ventilator 34, der an einer der Öffnungen 32 angeordnet ist.

Horizontal neben der ersten Kompensationskammer 30 ist eine zweite Kompensationskammer 36 angeordnet, die über verschließbare Öffnungen 37 mit dem Innenraum 20 des Raumes 10 kommunizieren kann. Auch hier ist eine der Verbindungsöffnungen 37 zur Verbesserung des Luftaustausches zwischen dem Innenraum 20 und der zweiten Kammer 36 mit einem Ventilator 39 ausgerüstet. Nach unten zum Innenraum 20 hin ist die zweite Kammer 36 durch eine Zwischendecke 38 begrenzt.

Die beiden als erste Kompensationskammer 30 und zweite Kammer 36 bezeichneten Beutel sind vorzugsweise aus Polyvinylfluorid hergestellt. Sie sind als etwa quaderförmige Beutel ausgestaltet, die zu Beginn eines jeden Tests mittels der genannten Ventilatoren 34, 39 jeweils prall mit Luft gefüllt werden. Hierdurch ist einerseits eine exakte Bestimmung des Ausgangsvolumens ohne Verwendung von Luftmengenmeßeinrichtungen möglich, und anderereseits ist mit dieser Einrichtung die Volumenänderung des Innenraums 20 zur Anpassung des Innendrucks an den Außendruck ohne zusätzliche Meß- und Regeleinrichtungen möglich. Dabei dient der größere Beutel, nämlich die erste Kompensationskammer 30 dazu, Volumenausdehnungen aufgrund von Temperaturerhöhung im Innenraum 20 sowie aufgrund der Abnahme des Umgebungsdrucks zu kompensieren, während der kleinere Beutel, nämlich die zweite Kammer 36, ausschließlich zur Kompensation von Umgebungsdruckerhöhungen und der hieraus resultierenden Volumenerweiterung des Innenraums 20 durch entsprechende Volumenverringerung der Kompensationskammer 30 dient.

Nachstehend soll die Wirkungsweise der erfindungsgemäßen Vorrichtung erläutert werden.

Während der Vorbereitungsphase, d. h. bis zum Zeitpunkt, bis das zu überprüfende Fahrzeug in dem Raum 10 eingestellt ist, sind die Klappen 37 in der zweiten Kammer 36 geöffnet sowie die Ventilatoren 39 an der zweiten Kammer sowie 34 an der ersten Kompensationskammer eingeschaltet. Außerdem ist der an der Heiz-und Kühleinrichtung angeordnete Spülluftventilator 25 eingeschaltet. Die Ventilatoren 34, 39 fördern jeweils Luft in die zugeordneten ersten und zweiten Kammern 30, 36 und sorgen dafür, daß diese ihre Ausgangsposition einnehmen. Diese Ausgangsposition ist dadurch gekennzeichnet, daß die die Kammern 30, 36 bildenden Beutel zwischen der Decke 12 und der zweiten Zwischendecke 28 eingepreßt sind. Nun kann das Prüfverfahren beginnen.

Zu Beginn des Prüfverfahrens wird zunächst das Tor 16 geschlossen. Der außenliegende Ventilator 34, der in die erste Kompensationskammer 30 Luft fördert, wird ausgeschaltet, während der an der zweiten Kammer 36 angeordnete Ventilator 39 weiterläuft, um die zweite Kammer 36 zu durchlüften. Hierbei kann gegebenenfalls auch eine niedrigere Drehzahl vorgesehen sein.

Zu diesem Zeitpunkt wird die Kammertemperatur auf das Minimum von 18 bis 21°C eingestellt. Anschließend wird die Kammertemperatur in sinusförmigen Zyklus temperatur-zeit-abhängig auf einen Temperaturverlauf zwischen 18 und 41°C geregelt. Die hieraus resultierende Druckerhöhung im Innenraum 20 gegenüber dem Umgebungsdruck wird dadurch kompensiert, daß sich das Volumen der ersten Kammer 30 verringert, indem die Innenluft durch die Durchtrittsöffnungen 27, 29 in der ersten und zweiten Zwischendecke 26, 28 hindurchtritt und die Beutelwand der ersten Kammer 30 beaufschlagt. Die hierdurch erforderliche Volumenänderung kann bis zu ca. 8 % des gesamten Volumens des Innenraums 20 betragen.

Sollte während dieser Zeit der Außendruck abnehmen, so kann ein Druckausgleich dadurch erreicht werden, daß das Volumen der ersten Kompensationskammer 30 weiter verringert wird. Ein derartiger Druckabfall kann bis zu 4 % des Raumvolumens des Innenraums 20 betragen. Um diese zusätzliche Ausdehnung kompensieren zu können, wird als Volumen für die erste Kompensationskammer 30 ein Wert von ca. 15 % des Gesamtvolumens des Innenraums 20 zugrundegelegt.

Für den Fall, daß der Außendruck während des 72 Stunden dauernden Prüfzyklusses zunimmt und hierdurch eine Komprimierung der Luftmenge innerhalb der ersten Kompensationskammer 30 hervorruft, erfolgt die erforderliche Druckkompensation dadurch, daß das Volumen der zweiten Kammer 36 verringert wird. Das Kammervolumen der zweiten Kammer 36 wird zu diesem Zweck auf ca. 5 % des Gesamtvolumens ausgelegt, wobei die erwähnt Komprimierung bis 4 % des Kammervolumens betragen kann.

Mit Hilfe der erfindungsgemäßen Vorrichtung kann der Rauminnendruck so geregelt werden, daß Druckdifferenzen im Vergleich zum Umgebungsdruck praktisch zu Null werden. Die maximale Druckdifferenz wird nur bestimmt durch das Eigengewicht und die Steifheit der verwendeten Folie für die beiden Kammern 30, 36 sowie durch die von dem an der zweiten Kammer 36 angeordneten Ventilator 39 erzeugten Druckdifferenz.

Als besonderer Vorteil des erfindungsgemäßen Verfahrens sowie der hierzu vorgesehenen Vorrichtung ist hervorzuheben, daß seine Durchführung gänzlich ohne Steuersystem und bei minimaler Druckdifferenz zur Umgebung völlig selbsttätig erfolgt.

Anstelle der in der einzigen Figur gezeigten Anordnung der ersten und zweiten Kompensationskammern 30, 36 innnerhalb des Raums 10 kann die erste Kompensationskammer 30 auch in einem Außenraum angeordnet sein, der gegenüber dem Raum 10 abgetrennt ist, wobei allerdings die darin angeordnete erste Kompensationskammer 30 über Zu- und Abluftöffnungen, die mit Ventilatoren bestückt sind, mit dem Raum 10 verbunden ist. Dieser sogenannten Außenraum besitzt Luftöffnungen entsprechend den Öffnungen 32, die die Verbindung zur Umgebung bilden.

## Patentansprüche

1. Verfahren zur Erfassung der Verdampfungsemission eines Fahrzeugs (18), insbesondere zur Erfassung dessen Emission von Kohlenwasserstoffverbindungen, dadurch gekennzeichnet, daß das Fahrzeug (18) in einem gasdicht abschließbaren Raum (10) abgestellt wird, daß entsprechend einer vorgegebenen Temperatur-Zeit-Kurve die im Raum (10) befindliche Luft zyklisch erwärmt und abgekühlt wird, daß die während dieser Zyklen emittierten Kohlenwasserstoffverbindungen qualitativ und quantitativ bestimmt werden und daß der Innendruck in dem Raum (10) konstant gehalten wird.

2. Verfahren nach Anspruch 1, dadurch gekennzeichnet, daß der Innendruck in dem Raum (10) durch Änderung seines freien Volumens an den Umgebungsaußendruck angepaßt wird.

3. Verfahren nach Anspruch 2, dadurch gekennzeichnet, daß zur veränderlichen Einstellung des Volumens des Raums (10) je ein erster und ein zweiter Teil des Raumvolumens abgetrennt werden, deren Volumen entsprechend dem jeweiligen Druck vergrößert oder verkleinert wird, so daß das Freiraumvolumen in dem Raum (10) sich ebenfalls entsprechend verringert oder vergrößert.

4. Vorrichtung zur Durchführung des Verfahrens nach einem der Ansprüche 1 bis 3, dadurch gekennzeichnet, daß ein gasdicht abschließbarer Raum (10) zur Aufnahme eines Fahrzeugs 18 mit einer Heiz- und Kühlvorrichtung (24) zur Einstellung eines bestimmten Temperatur-Zeit-Verlaufs sowie mit einer Gaserfassungseinrichtung versehen ist und daß in dem Raum eine erste gasdicht abgetrennt Kompensationskammer (30) angeordnet ist, welche mit der Umgebungsatmosphäre oder mit einem Außenraum in Verbindung steht, sowie eine zweite Kammer (36), die über verschließbare Öffnungen (37) mit dem Innenraum (20) des Raums (10) in Verbindung steht.

5. Vorrichtung nach Anspruch 4, dadurch gekennzeichnet, daß die erste und die zweite Kammer (30, 36) mit Ventilatoren (34, 39) versehen sind, über welche die Kammern (30, 36) mit Luft befüllbar oder entleerbar sind.

6. Vorrichtung nach einem der vorherigen Ansprüche, dadurch gekennzeichnet, daß die Kammern (30, 36) als flexible Beutel ausgebildet sind.
